# FASCICULE DE BREVET EUROPEEN

(11) **EP 1 012 239 B1**
(45) Date de publication et mention de la délivrance du brevet: **25.11.2009**
(21) Numéro de dépôt: 99911899.5
(22) Date de dépôt: 08.04.1999
(51) Int. Cl.: C12N 5/08, A61K 35/14

(54) **PROCEDE ET SYSTEME DE GESTION DE LOTS DE CELLULES IMMUNO-COMPETENTES EN VUE D'UTILISATIONS DIFFEREES**
VERFAHREN UND SYSTEM ZUR VERWALTUNG VON GRUPPEN IMMUNKOMPETENTER ZELLEN ZUR SPÄTEREN VERWENDUNG
METHOD AND SYSTEM FOR MANAGING IMMUNOCOMPETENT CELL BATCHES FOR DELAYED USE

(30) Priorité: 08.04.1998 WO PCT/FR98/00708
(43) Date de publication de la demande: 28.06.2000
(73) Titulaire: Lefesvre, André, 75008 Paris (FR); Sankhia International Inc, Road Town, Tortola (VG)
(72) Inventeur: Lefesvre, André, 75008 Paris (FR)
(74) Mandataire: Pontet, Bernard
(86) Numéro de dépôt international: PCT/FR1999/000810
(87) Numéro de publication internationale: WO 1999/053030

(56) Documents cités:
- WO-A-89/04168
- WO-A-94/00567
- DE-A- 3 421 011

## Description

La présente invention concerne un procédé de gestion de lots de cellules de lymphocytes ou de monocytes, en vue d'utilisations différées.

Des travaux scientifiques et cliniques ont mis en évidence les vertus thérapeutiques de l'auto-utilisation de dérivés lymphocytaires et monocytaires qui contribue notamment à une augmentation de l'immunité cellulaire.

Une application prometteuse de cette méthode thérapeutique concerne la possibilité de renforcer l'immunité d'un patient à un moment de sa vie où ce renforcement s'avère nécessaire ou vital ou pour maintenir cette immunité au fil de sa vie.

Cependant, une difficulté importante à surmonter réside dans la disponibilité des lymphocytes ou monocytes d'un patient sur des périodes de temps qui pourraient être comprises entre plusieurs mois et plusieurs dizaines d'années. On connaît déjà des techniques de stockage cryogénique désormais largement utilisées dans de nombreux domaines de la biologie humaine et animale. Des banques ont notamment été mises en place pour la conservation et le stockage d'éléments biologiques.

Le document WO89/04168 divulgue un procédé d'isolement et de conservation de cellules hématopoïétiques de sang foetal et néonatal. Ce procédé vise notamment l'utilisation thérapeutique de cellules foetales et néonatales pour des reconstitutions hématopoïétiques ou en thérapie génique, et peut être mis en oeuvre pour la cryo-conservation de cellules de sang foetal ou néonatal à des fins de reconstitution autologue.

Le document WO 94/00567 décrit une méthode pour stabiliser des lymphocytes et/ou des cellules souches prélevées sur un mammifère, dans un milieu aqueux comprenant de la gélatine. Lors de leur stockage, les cellules sont maintenues à une température inférieure à 8°C, mais pas en dessous de la température de gel.

Après stockage, les cellules peuvent être administrées, après chauffage, sans autre traitement.

Le document WO 91/09521 décrit un procédé de préparation d'échantillons biologiques consistant, après la naissance d'un individu, à prélever un segment de cordon ombilical, à le congeler et à enregistrer des données sur l'individu, puis à transférer le segment ombilical vers un site de stockage cryogénique.

Le document décrit également le traitement thérapeutique d'un individu avec un composant isolé à partir de segment de cordon ombilical qui a été stocké.

Les cellules immuno-compècentes (lymphocytes, cellules phagocytaires : monocytes, macrophages) jouent un rôle central dans le système immunitaire. En particulier, les lymphocytes stockent des informations au cours de la vie et sont le support de la mémoire de l'immunité cellulaire et humorale.

Cette bibliothèque, lymphocytaire en particulier, mais aussi l'ensemble du système immunitaire, s'enrichit au cours de la vie, au fur et à mesure que l'individu rencontre des organismes étrangers, lors des infections virales, parasitaires ou bactériennes. C'est grâce à elle que l'organisme peut minimiser l'impact des infections au cours de la vie. Le mécanisme d'action de l'immunité cellulaire est connu. Les informations sont notamment stockées dans les parois des lymphocytes, comme le montre le facteur de transfert et de nombreuses publications. Ce mécanisme contribue également à la défense contre les cellules malignes.

D'une part, cette mémoire s'efface en partie avec le temps, comme le montre la nécessité de pratiquer des rappels de vaccination pour conserver une protection efficace. En ce qui concerne l'immunité humorale, les taux d'anticorps baissent, rapidement pour les IgM, plus lentement pour les IgG et les IgA.

D'autre part, des erreurs s'introduisent avec le temps et l'immunité devient souvent moins efficace avec les années. C'est à cause de cette dégradation que des infections comme la grippe sont beaucoup plus graves chez les personnes âgées. Il y aurait donc intérêt à pouvoir conserver les informations acquises tout au cours de la vie.

Les prélèvements peuvent avoir lieu à tout âge, dès que le système immunitaire est mature, soit par prise de sang périphérique, soit par leucophérèse, soit par prélèvement de moelle. Ces prélèvements conservent une information plus importante, puisqu'il s'agit de cellules complètes et vivantes, que celle transmise par le facteur de transfert qui n'est qu'un extrait de paroi lymphocytaire. La valeur non négligeable du facteur de transfert est largement prouvée.

Or, il s'avère que les infrastructures et méthodes actuelles en matière de stockage et de gestion de lots biologiques ne sont pas adaptées à l'application spécifique de l'auto-injection différée à long terme qui nécessite une gestion à très long terme des lots de cellules immuno-compétentes.

Le but de l'invention est de proposer un procédé pour gérer des lots de cellules de lymphocytes et/ou de monocytes, qui permette de conserver les informations acquises tout au cours de la vie, avec une garantie totale de stockage et de disponibilité à très long terme.

Cet objectif est atteint grâce au procédé selon la revendication 1.

Le procédé de gestion selon l'invention peut en outre comporter un plan de prélèvement des lots de cellules de lymphocytes et/ou monocytes à différentes étapes de la vie de chaque sujet humain bénéficiant de ce procédé.

Ce plan de prélèvement définit des étapes de prélèvement à des âges prédéterminés du sujet humain, ainsi que des étapes de prélèvement non programmées conditionnées par des événements particuliers de la vie du sujet humain.

Ainsi, avec le procédé selon l'invention, il devient possible de préserver le capital immunitaire et génétique de toute altération au cours de la vie d'un individu en réalisant une conservation individuelle d'éléments immuno-compétents avec la faculté d'en faire, si on le désire, une culture avec une croissance.

Il est en effet possible de décongeler une partie aliquote des cellules de lymphocytes et/ou monocytes conservées, de les cultiver, de stimuler leurs fonctions par exemple avec l'interleukine 2 ou l'interleukine 12.

La conservation de ces cellules permet également une analyse génétique différée qui, par comparaison, peut révéler des mutations survenues entre temps. La conservation du plasma peut également permettre de retrouver la trace d'infections inconnues au moment du prélèvement.

Dans un mode préféré de mise en oeuvre du procédé selon l'invention, chaque lot de lymphocytes associé à un sujet est conditionné et stocké dans un site de stockage cryogénique parmi une pluralité de sites de stockage cryogénique, puis transféré à la demande depuis ce site de stockage vers un centre de traitement cellulaire pour être réutilisé, et à chaque phase de prélèvement est associé une collecte, d'une part, de données personnelles relatives au sujet prélevé et d'autre part, de données relatives au prélèvement.

Ainsi, un tel procédé apporte aux patients la garantie d'une conservation de leurs lymphocytes sur le long terme, avec la perspective de pouvoir en disposer à tout moment pour entre autre renforcer leur système immunitaire. Il devient alors possible de redonner aux personnes leur immunité antérieure et de transmettre une immunité cellulaire, dans des conditions de gestion rationnelle et fiable, et en outre d'avoir accès à leur code génétique correspondant à la date de prélèvement du sang.

De préférence, à l'issue d'une phase de prélèvement, les lymphocytes prélevés sont conditionnés sous la forme d'une pluralité de lots de lymphocytes. Ceci permet une gestion souple et efficace des réinjections, sans crainte de devoir décongeler un lot de lymphocytes en quantité excessive par rapport aux besoins ponctuels.

Une mise en oeuvre avantageuse du procédé selon l'invention consiste en ce que les lots de cellules de lymphocytes et/ou monocytes sont stockés dans plusieurs sites de stockage cryogénique géographiquement distincts. Cette caractéristique vise à augmenter la sécurité de conservation des lymphocytes ou monocytes prélevés. Plus généralement, dans le procédé de gestion selon l'invention, la notion d'utilisation différée recouvre à la fois le domaine des auto-utilisations, d'utilisation de facteur de transfert obtenus à partir des prélèvements lymphocytaires, ou d'utilisation des monocytes, ou d'utilisation de culture de lymphocytes avec des facteurs de croissance tels que l'interleukine 2, et le domaine des utilisations de filiation, notamment en thérapie génique, notamment la lecture du code génétique.

Les lots de lymphocytes ou de monocytes peuvent par exemple être réutilisés après culture cellulaire en présence de médiateurs cellulaires, tels que l'interleukine 2.

D'autres particularités et avantages de l'invention apparaîtront encore dans la description ci-après. Aux dessins annexés donnés à titre d'exemples non limitatifs:
- la figure 1 est un schéma synoptique du procédé et du système de gestion de prélèvements lymphocytaires selon l'invention;
- la figure 2 illustre les flux respectifs de prélèvements, de données personnelles et de données de gestion, résultant de la mise en oeuvre du procédé de gestion selon l'invention; et
- la figure 3 illustre les relations entre sites de réinjection, de stockage et de gestion, au sein d'un système de gestion selon l'invention.

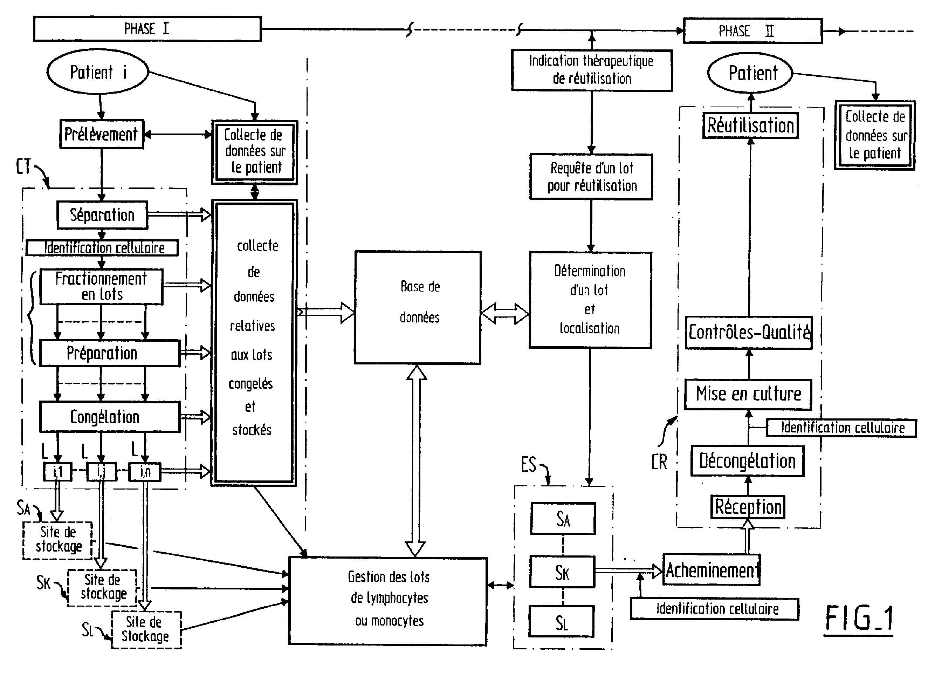

On va maintenant décrire un exemple de mise en oeuvre du procédé de gestion de prélèvements lymphocytaires ou monocytaires, en même temps que le système de gestion correspondant, en référence à la figure 1.

Une première phase I concerne le prélèvement et la réalisation des lots lymphocytaires ou monocytaires.

Les étapes suivantes sont effectuées lors d'une phase I réalisée dans un centre spécialisé CT:
- une collecte de données, notamment des données relatives au groupage tissulaire, et d'informations relatives à un patient i,
- un prélèvement de sang sur ce patient,
- un traitement du sang prélevé et une séparation des lymphocytes et/ou monocytes,
- une identification cellulaire,
- un fractionnement pour réaliser un ensemble de lots de lymphocytes et/ou monocytes,
- une préparation des lymphocytes et/ou monocytes, incluant entre autre, éventuellement une déshydratation,
- une congélation et mise en stockage cryogénique de n lots de lymphocytes et/ou monocytes L_{i,1,} L_{i,j}, L_{i,n}, dans un système de réfrigération approprié, par exemple dans une atmosphère gazeuse de réfrigération.

Il est à noter que les opérations de séparation et de fractionnement peuvent être intégrées et réalisées au sein d'un appareil à cytophérèse. Par ailleurs, l'étape d'identification cellulaire, qui peut mettre en oeuvre diverses techniques connues d'identification, peut intervenir à d'autres stades de la phase I en fonction de spécificités techniques.

Les lots de lymphocytes et/ou monocytes congelés sont ensuite répartis dans différents sites ou banques de stockage S_{A}, S_{K}, S_{L}. Les stockages de lots de lymphocytes sont sécurisés et gérés. Cette gestion met en oeuvre une base de données alimentées par des données collectées lors de chaque phase de prélèvement et de stockage I. Les lots de cellules pourraient ainsi être stockés sur des durées très variables pouvant aller de quelques jours à plusieurs dizaines d'années sous réserve de la garantie d'une bonne conservation des lymphocytes ou monocytes sur le long terme. Par ailleurs, le principe de ne pas stocker l'ensemble des lots d'un patient dans un même site contribue amplement à la sécurité d'approvisionnement.

Lorsqu'une indication thérapeutique concernant un patient ayant bénéficié de ce procédé de gestion préconise une utilisation de lymphocytes ou monocytes, on entreprend alors la phase II du procédé. La société gestionnaire du procédé est contactée et reçoit une requête pour un lot de cellules stocké et géré pour le compte de ce patient. Grâce à l'interrogation de la base de données, on détermine et on localise un lot appartenant à ce patient dans l'un des sites de stockage S_{A}, S_{K}, S_{L} de l'ensemble de sites ES. Après localisation et identification cellulaire, le lot concerné est acheminé par transport rapide jusqu'à un centre de traitement cellulaire CR qui peut d'ailleurs être le centre dans lequel le prélèvement initial avait été effectué.

La phase de réutilisation (par exemple, réinjection) comprend par exemple:
- une réception du lot incluant un contrôle d'identification qui peut notamment comporter une lecture d'un code-barre et un groupage tissulaire,
- une décongélation du lot,
- une nouvelle identification cellulaire,
- une remise en suspension des lymphocytes ou monocytes dans un milieu de culture,
- des opérations de contrôle de qualité et de sécurité, et
- une réutilisation des lymphocytes dans le patient.

A l'occasion de cette phase II de réutilisation, de nouvelles données relatives au patient peuvent à nouveau être collectées à des fins d'études et de traitement statistiques.

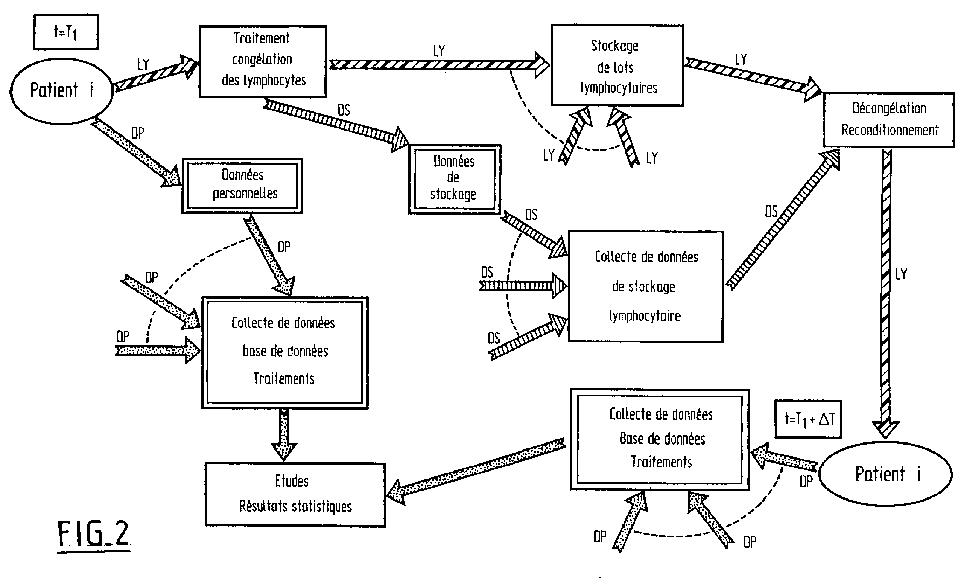

On va maintenant décrire le procédé de gestion en termes de flux, avec une analyse des transferts d'éléments biologiques, de données personnelles et de données relatives aux prélèvements et stockages, en référence à la figure 2. A partir de tout patient faisant appel à un instant T1 aux services d'une société de gestion de prélèvement, sont effectués les transferts suivant:
- un transfert d'éléments biologiques constitués par les lymphocytes LY ou monocytes prélevés et séparés,
- un transfert de données personnelles DP collectées à l'occasion des prélèvements, sous réserve du respect des dispositions légales propres à chaque état,
- un transfert de données et paramètres directement associés aux prélèvements et indispensables pour la gestion des stocks de prélèvement.

Chacun de ces types de transfert conduit à des processus de collecte:
- une collecte des lots de cellules avec une gestion centralisée et des sites de stockage diversifiés,
- une collecte des données personnelles pour alimenter des bases de données statistiques, et
- une collecte des données de prélèvements pour constituer une base de données de gestion des lots de cellules.

Lorsqu'une phase II de réutilisation, par exemple sous la forme d'une réinjection, est décidée à un instant T1+ΔT, sont réalisés les transferts suivants:
- un transfert d'un lot de cellules depuis un site de stockage vers un centre de traitement cellulaire,
- un transfert des données et paramètres associés à ce lot, et
- un transfert de données personnelles réactualisées sur ce patient, qui sont collectées à l'occasion de la phase de réutilisation.

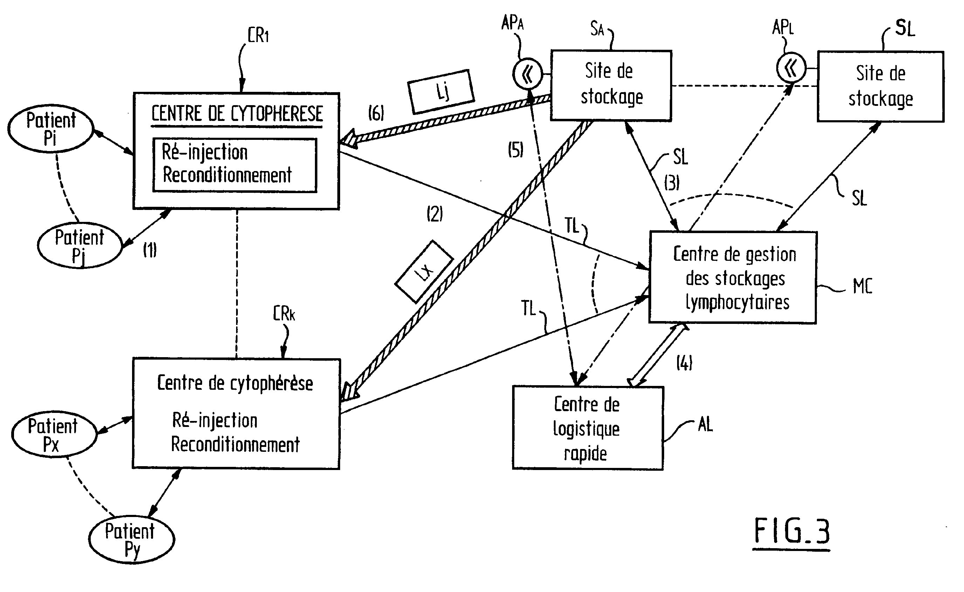

On va maintenant décrire les conditions de réalisation d'une phase de réutilisation dans le cadre du procédé de gestion selon l'invention, plus particulièrement en termes de localisation géographique et de logistique, en référence à la figure 3.

Sur un territoire donné, qui peut être un continent, un état, une région, ou une communauté urbaine, des centres de cytophérèse ou de thérapie lymphocytaire CR₁, CR_{K} sont mis en place. Ces centres, prévus pour recevoir des patients Pi, Pj, Px, Py ayant adhéré à un programme de prélèvement-réutilisation mettant en oeuvre le procédé de gestion selon l'invention, sont en communication par des moyens de liaison TL tels que des réseaux de communication fermés ou ouverts (Internet) à un ou des centres MC de gestion des stockages de lymphocytes ou monocytes qui peuvent être localisés en tout point du globe. Ce centre de gestion MC est en communication permanente par des moyens de communication appropriés SL (liaisons spécialisées ou réseau ouvert tel qu'Internet) avec l'ensemble des sites de stockage S_{A}, .., S_{L}. Par ailleurs, le centre de gestion MC coopère avec un centre de logistique rapide AL qui pilote un ensemble de plates-formes d'expédition rapide AP_{A}, AP_{L} à proximité desquelles les sites de stockage sont de préférence implantés. A titre d'exemple de mise en oeuvre du procédé de gestion selon l'invention, un patient j contacte (1), sur indication médicale, un centre de cytophérèse ou réutilisation CR₁ de son choix, par exemple le centre le plus proche de chez lui. Le centre de traitement cellulaire CR₁ consulte sur un terminal connecté au centre de gestion MC et lui transmet (2) une requête de lot. Le centre de gestion MC interroge la base de données de gestion et localise un lot de cellules Lj correspondant à ce patient dans l'un des sites de stockage. En cas de stockage multiple diversifié, le site de stockage le plus proche du centre de traitement cellulaire CR₁ est choisi. Le centre de gestion MC transmet (3) la requête au site de stockage sélectionné S_{A} et contacte (4) le centre le logistique rapide AL. Le site de stockage S_{A} extrait alors le lot concerné et le transmet immédiatement à la plate-forme d'expédition rapide AP_{A} qui a été préalablement activée (5) par le centre de logistique rapide AL. Le lot Lj est alors acheminé (6) par les moyens rapides disponibles, de préférence par avion, à destination du centre de traitement cellulaire CR₁.

Il est à noter que chaque patient adhérant à un tel programme dispose généralement d'un stock de lots de cellules qui permet par exemple d'étaler dans le temps des auto-utilisations successives, par exemple sous la forme d'auto-injections, à des fins de renforcement du système immunitaire ou de thérapie génique ou autre, ou encore d'utiliser massivement en cas de besoin le stock de lymphocytes ou monocytes ainsi constitué.

Le procédé de gestion selon l'invention est de préférence matérialisé sous la forme d'un logiciel implanté sur des systèmes de gestion et de traitement d'information qui peuvent être localisés dans des centres de gestion de lots et être reliés à un ensemble de sites informatiques locaux au sein des centres de cytophérèse, de logistique rapide et de stockage.

Bien sûr, l'invention n'est pas limitée aux exemples qui viennent d'être décrits et de nombreux aménagements peuvent être apportés à ces exemples sans sortir du cadre de l'invention. Ainsi, on peut prévoir, dans le cadre des phases de prélèvement et de réutilisation, des étapes techniques supplémentaires en fonction d'impératifs médicaux et de contraintes de sécurité. Les moyens de communication mis en oeuvre entre les divers centres opérationnels et de gestion peuvent être de toute nature.

Par ailleurs, les informations collectées dans le cadre du procédé de gestion selon l'invention peuvent être avantageusement traitées à des fins statistiques, avec des applications dans le domaine de la prévention et de l'assurance.

## Revendications

1. Procédé pour gérer des lots de cellules de lymphocytes et/ou de monocytes en vue d'une utilisation différée, ces lots provenant de sang ayant été prélevé sur un patient lors de phases successives de prélèvement echelonnées durant la vie de patient, chaque phase de prélèvement comprenant en outre, les étapes suivantes:
a. une collecte de données, notamment des données relatives au groupage tissulaire, et d'informations relatives à un patient,
b une séparation des lymphocytes et/ou monocytes du sang prélevé,
c. une identification cellulaire,
d. un fractionnement pour réaliser un ensemble de lots de lymphocytes et/ou monocytes,
e. une préparation des lymphocytes et/ou monocytes, incluant entre autre, éventuellement une déshydratation,
f. une congélation et mise en stockage cryogénique d'au moins partie desdits lots de lymphocytes et/ou monocytes dans un système de réfrigération approprié, dans un ou plusieurs sites de stockage ; et le procédé comprenant en outre :
- au moins une phase de réutilisation, effectuée lorsqu'une indication thérapeutique concernant le patient ayant fait l'objet des prélèvements ci-dessus préconise une utilisation de lymphocytes et/ou monocytes, ladite phase de réutilisation comprenant les étapes suivantes :
g. interrogation de la base de données pour déterminer et localiser un lot appartenant au patient cl-dessus dans l'un desdits sites de stockage,
h. après localisation et identification cellulaire, acheminement du lot concerné dudit site de stockage jusqu'à un centre de traitement cellulaire,
i. réception dudit lot identifié et contrôle d'identification tel qu'une lecture d'un code-barre et d'un groupage tissulaire,
j. décongélation du lot,
k. nouvelle identification cellulaire,
l. remise en suspension des lymphocytes et/ou monocytes dans un milieu de culture,
m. contrôle de qualité et de sécurité, en vue d'une réutilisation différée des lymphocytes et/ou monocytes dans le même patient .

2. Procédé selon la revendication 1, **caractérisé en ce qu'**il comprend en outre un plan de prélèvement de lots de cellules de lymphocytes et/ou monocytes à différentes étapes de la vie d'un patient bénéficiant dudit procédé.

3. Procédé selon la revendication 2, **caractérisé en ce que** le plan de prélèvement qui définit des étapes de prélèvement à des âges prédéterminés du patient.

4. Procédé selon la revendication 3, **caractérisé en ce que** le plan de prélèvement qui définit en outre des étapes de prélèvement non programmées conditionnées par des événements particuliers de la vie du patient.

5. Procédé selon l'une quelconque des revendications précédentes, **caractérisé en ce que** des lots desdites cellules sont réutilisés pour produire des facteurs de transfert.

6. Procédé selon l'une quelconque des revendications précédentes, **caractérisé en ce que** les lots desdites cellules sont réutilisés après culture cellulaire en présence de médiateurs cellulaires, tels que l'interleukine 2.

7. Procédé selon l'une quelconque des revendications précédentes, **caractérisé en ce que** les lots desdites cellules sont réutilisés pour effectuer une analyse génétique différée.

8. Procédé selon l'une quelconque des revendications précédentes, **caractérisé en ce que** les lots desdites cellules sont réutilisés pour retrouver la trace d'infections inconnues au moment du prélèvement desdits lots.

## Claims

1. Process for management of batches of lymphocytes and/or monocytes cells in view of a deferred use, said batches being issued from blood collected from a patient during successives phases of collection spaced out during the patient's live, each collection phase comprising further, the following steps :
a) gathering of data, in particular data relating to the tissue group and information relating to a patient,
b) a separation of the lymphocytes and/or monocytes from the collected blood,
c) a cell identification,
d) a fractionation to obtain a group of lymphocytes and/or monocytes batches,
e) a preparation of the lymphocytes and/or monocytes including inter alia an eventual dehydration,
f) a freezing and cryogenic storage of at least a part of said lymphocytes and/or monocytes batches in a suitable refrigeration system, in one or several storage sites ; and the process further comprising : at least a re-use phase, effected when a therapeutic indication related to the patient having been subjected to above collections specifies the use of lymphocytes and/or monocytes, said re-use phase comprising the following steps :
g) interrogation of the data base to determine and locate a batch belonging to above patient in one of the said storage sites,
h) after location and cell identification, forwarding the concerned batch from the storage site to a cell treatment center,
i) receipt of said identified batch and identification control such as reading a bar-code and a tissue group,
j) thawing of the batch,
k) another cell identification,
l) resuspending the lymphocytes and/or monocytes in a culture medium,
m) quality and safety control in view of a deferred re-use of the lymphocytes and/or monocytes in the same patient.

2. Process according to claim 1, **characterized in that** it further comprises a collection plan of the batches of lymphocytes and/or monocytes cells at different steps of the live of the patient having the benefit of said process.

3. Process according to claim 2, **characterized in that** the collection plan defines collection steps at predetermined ages of the patient.

4. Process according to claim 3, **characterized in that** the collection plan further defines non programmed collection steps which results from particular events of the patient's live.

5. Process according to any of proceding claims, **characterized in that** the batches of said cells are re-used to produce transfer factors.

6. Process according to any of preceding claims, **characterized in that** the batches of said cells are re-used after cellular culture in the presence of cellular mediators, such as interleukine 2.

7. Process according to any of preceding claims, **characterized in that** the batches of said cells are re-used for effecting a deferred genetic analysis.

8. Process according to any of preceding claims, **characterized in that** the batches of said cells are re-used to find the trace of infections unknown at the moment of said batches collection.

## Patentansprüche

1. Verfahren zur Handhabung von Lymphozyt- und/oder Monozyt-Zellchargen zur Verwendung zu einem späteren Zeitpunkt, wobei die Konzentrate von Blut stammen, das einem Patienten im Laufe seines Lebens in aufeinander folgenden gestaffelten Phasen entnommen wurde, wobei jede Entnahmephase unter anderem die folgenden Schritte umfasst:
a) Datenerfassung, insbesondere von die Gewebegruppierung betreffenden Daten und patientenbezogenen Informationen,
b) Absondern der Lymphozyten und/oder Monozyten aus dem entnommenen Blut,
c) Zellidentifikation,
d) Fraktionierung zur Gewinnung einer Gesamtmenge an Lymphozyt-und/oder Monozyt-Chargen,
e) Vorbereitung der Lymphozyten und/oder Monozyten, umfassend bei Bedarf unter anderem eine Dehydrierung,
f) Einfrierung und Tieftemperaturlagerung von zumindest einem Teil der Lymphozyt- und/oder Monozyt-Chargen in einem geeigneten Kühlsystem an einem oder an mehreren Lagerungsorten, wobei das Verfahren unter anderem Folgendes umfasst:
- zumindest eine Wiederverwendungsphase, die dann durchgeführt wird, wenn bei dem Patienten, bei dem die vorgenannten Entnahmen vorgenommen wurden, eine therapeutische Indikation vorliegt, die eine Verwendung von Lymphozyten und/oder Monozyten nahe legt, wobei die Wiederverwendungsphase die folgenden Schritte umfasst:
g) Abfrage der Datenbank zur Bestimmung und Lokalisierung einer dem betreffenden Patienten zugeordneten Charge an einem der Lagerungsorte,
h) im Anschluss an die Lokalisierung und Zellidentifikation Überführen der entsprechenden Charge von dem Lagerungsort an ein Zellbehandlungszentrum,
i) Entgegennahme der identifizierten Charge und Identifikationskontrolle z. B. durch Ablesen von einem Barcode und einer Gewebegruppierung,
j) Auftauen der Charge,
k) erneute Zellidentifikation,
l) Suspendieren der Lymphozyten und/oder Monozyten in einem Nährmedium,
m) Qualitäts- und Sicherheitskontrolle zwecks Wiederverwendung der Lymphozyten und/oder Monozythen zu einem späteren Zeitpunkt bei demselben Patienten.

2. Verfahren nach Anspruch 1,
**dadurch gekennzeichnet,**
**dass** es zudem einen Plan für die Entnahme der Lymphozyt-und/oder Monozyt-Zellchargen in unterschiedlichen Lebensphasen eines von diesem Verfahren profitierenden Patienten umfasst.

3. Verfahren nach Anspruch 2,
**dadurch gekennzeichnet,**
**dass** der Entnahmeplan Entnahmephasen in bestimmten Alterstufen eines Patienten vorsieht.

4. Verfahren nach Anspruch 3,
**dadurch gekennzeichnet,**
**dass** der Entnahmeplan ferner nicht festgelegte, durch besondere Vorkommnisse im Leben des Patienten bedingte Entnahmephasen vorsieht.

5. Verfahren nach einem der vorangehenden Ansprüche,
**dadurch gekennzeichnet,**
**dass** die Chargen dieser Zellen zur Erzeugung von Transferfaktoren wiederverwendet werden.

6. Verfahren nach einem der vorangehenden Ansprüche,
**dadurch gekennzeichnet,**
**dass** die Chargen dieser Zellen nach der Zellkultivierung in Gegenwart von zellulären Botenstoffen, wie beispielsweise Interleukin-2, wiederverwendet werden.

7. Verfahren nach einem der vorangehenden Ansprüche,
**dadurch gekennzeichnet,**
**dass** die Chargen dieser Zellen zum Durchführen einer Genanalyse zu einem späteren Zeitpunkt wiederverwendet werden.

8. Verfahren nach einem der vorangehenden Ansprüche,
**dadurch gekennzeichnet,**
**dass** die Chargen dieser Zellen zum Aufspüren von unbekannten Infektionen zum Zeitpunkt der Entnahme dieser Chargen wiederverwendet werden.
